# EUROPEAN PATENT SPECIFICATION

(11) **EP 0 874 591 B1**
(45) Date of publication and mention of the grant of the patent: **06.04.2011**
(21) Application number: 96945202.8
(22) Date of filing: 05.12.1996
(51) Int. Cl.: A61B 17/00

(54) **VASCULAR WOUND CLOSURE DEVICE**
VORRICHTUNG ZUM SCHLIESSEN VON GEFÄSSWUNDEN
DISPOSITIF POUR LA FERMETURE DE PLAIES VASCULAIRES

(30) Priority: 07.12.1995 US 9643 P; 05.12.1996 US 764611
(43) Date of publication of application: 04.11.1998
(73) Proprietor: LOMA LINDA UNIVERSITY MEDICAL CENTER, Loma Linda, CA 92350 (US)
(72) Inventor: ZHU, Yong Hua, Redlands,CA 92373 (US); KIRSCH, Wolff, M., Redlands, CA 92373 (US)
(74) Representative: Marsh, Roy David
(86) International application number: PCT/US1996/019937
(87) International publication number: WO 1997/020505

(56) References cited:
- US-A- 1 064 307
- US-A- 3 888 117
- US-A- 5 104 394

## Description

### Field of the Invention

The present invention relates to a device to assist in the closure of puncture or other wounds in the vasculature of a patient. Specifically, the invention relates to a vascular wound closure device which aids in locating and isolating the hole in the vasculature and guiding an appropriate wound closure device to the site, so that the wound may be closed using surgical clips, sutures, or staples.

### Background of the Invention

Transluminal balloon angioplasty is used in the treatment of peripheral vascular disease to increase or restore blood flow through a significantly narrowed artery in a limb; it is also used in the treatment of blockage of the coronary arteries. In fact, coronary angioplasty has emerged as a major viable alternative to bypass surgery for revascularization of stenotic and occluded coronary arteries. Unlike bypass surgery, angioplasty does not require general anesthesia, opening of the chest wall, use of a heart-lung machine, or transfusion of blood. Angioplasty is not only less invasive and less traumatic to the patient, it is also less expensive because of the shorter hospital stay and shorter recovery time.

Transluminal balloon angioplasty is performed by first inserting a hollow needle through the skin and into the patient's femoral artery. A guidewire is advanced through the hollow needle and into the artery, then along the patient's vasculature toward the site of the blocked blood vessel or heart valve to be treated. X-ray imaging is used to help move the guidewire through the vascular system and into position just past the stenosis to be treated. A balloon catheter is then threaded over the guidewire and advanced until the deflated balloon is within the stenosis. The balloon is then repeatedly inflated to widen the narrowed blood vessel. After the procedure is complete, the catheter and guidewire are withdrawn from the blood vessels and the patient.

Angiography, which is used to detect diseases that alter the appearance of blood vessels, is performed in a similar manner. A hollow needle is first inserted through the skin and into the femoral artery, and a guidewire is then inserted through the needle and into the affected blood vessel. A catheter is then threaded over the guidewire and into the blood vessel to be examined, using x-ray imaging to guide the catheter to the desired position. Contrast medium is then injected, and a rapid sequence of x-ray pictures are taken so that blood flow along the affected vessel can be studied. Once complete, the catheter and guidewire are removed from the patient's body.

After the catheter and guidewire used during angioplasty or angiography are removed, the puncture wound in the femoral artery must be closed and the bleeding through the puncture site in the artery stopped. Currently, ice packs and pressure are applied to the artery for a period lasting up to several hours in an attempt to stop the bleeding. Although efforts have been made to close the puncture wound using staples, clips, and sutures, they have been unsuccessful, largely due to the inability to clearly locate and visualize the puncture wound in the femoral artery.

Other wounds in the vasculature of a patient can also be difficult to locate and access. Thus, a device and method to facilitate the closure wounds in the vasculature of a patient, such as femoral artery puncture wounds following transluminal balloon angioplasty and angiography, would be extremely beneficial. A device having the ability to aid in locating the puncture wound and facilnating the closure of the wound using staples, clips, or sutures would eliminate the prolonged bleeding currently associated with such wounds. EP0646350 discloses an introduction device for introduction through the skin and the wall of a blood vessel. The device includes a supply means for a haemostatic pharmacon.

### Summary of the invention

The invention is defined in claim 1 below. The dependent claims are directed to optional and preferred features.

The wound closure device of the present invention aids in locating and isolating a puncture wound in the vasculature of a patient. It is used in conjunction with a guidewire which is normally inserted into the vasculature during diagnostic and therapeutic procedures. The device of the present invention aids the physician in closing the wound, thus eliminating prolonged bleeding associated with these procedures.

In accordance with one embodiment of the present invention, the device comprises a body portion has a tapered distal end.

In one embodiment of the present invention, the device further comprises at least one handle located on the proximal end of the body portion and extending laterally from the body portion. The body portion and the cap are preferably comprised of a biocompatible engineering polymer, such as polypropylene, polyethylene, or polyterephthalate. The body portion and the cap can also be comprised of an elastomer. Most preferably, the body portion and the cap are comprised of a metal, such as stainless steel

In another embodiment of the present invention, the device further comprises a guidewire. The guidewire is inserted through the channel in the body portion and through the hole in the cap. Preferably, the guidewire has an inflatable balloon attached near its distal and.

In accordance with another embodiment of the present invention, there is provided a system for facilitating the closure of wounds in the vasculature of a patient. The system comprises a retractor having the body portion and the internally threaded cap of the invention defined in claim 1.

The system further comprises a guidewire having an inflatable balloon attached thereto. The guidewire is inserted through the channel in the body portion and through the hole in the cap. A surgical clip applicator having a distal end fitted with two laterally protruding wings is also used. The wings are adapted to fit within the channel in the body portion of the retractor. The surgical clip applicator further comprises a guide attached to the distal and of the applicator and extending laterally therefrom. The guide is adapted to receive the guidewire through its ends.

There is described a method for facilitating the closure of a wound in the vasculature of a patient. The method may comprise the steps of inserting a guidewire having a proximal end and a distal end and an inflatable balloon into the vasculature through the wound, until the distal end of the guidewire is within the vasculature and the proximal end remains outside the patient's body; inflating the inflatable balloon; withdrawing the proximal end of the guidewire from the patient's body until the inflated balloon is located in the vasculature at the site of the wound; and inserting the proximal end of the guidewire into a distal end of a retractor. The proximal and of the guidewire may be inserted in the retractor until the proximal end emerges through a hole in the cap. The method may further comprise the steps of advancing the retractor along the guidewire and into the patient's body until the distal tip at the retractor contacts the inflated balloon; removing the cap from the body portion of the retractor, separating the two balves of the retractor until the wound is visible; and closing the wound. The closing step may prererably comprise either clipping, stapling, or suturing the wound.

In a described method, a surgical clip applicator may be inserted into the retractor body portion after the cap is removed. The clip applicator may be advanced through the channel in the body portion until the applicator contacts the wound. The closing step may preferably comprise applying at least one surgical clip to the wound.

The method may preferably further comprise the step of deflating the balloon and withdrawing the guidewire from the patient's body prior to the closing step.

There is described another method for facilitating the closure of wounds in the vasculature of a patient. This method may comprise the steps of inserting a guidewire having a proximal end and a distal end into the vasculature through the wound, until the distal end of the guidewire is within the vasculature and the proximal end remains outside the patient's body; inserting a catheter having a proximal end and a distal and and having an inflatable balloon attached at the distal and, into the vasculature over the guidewire until the distal end is within the artery and the proximal end remains outside the patient's body; inflating the inflatable balloon: withdrawing the catheter tram the patient's body until the inflated balloon is located in the vasculature at the site of the wound; and inserting the proximal end of the guidewire and catheter into the distal end of a retractor. The guidewire and catheter may be advanced as a unit until the proximal and of the guidewire emerges through the hole in the cap of the retractor.

The method may further comprise the steps of advancing the retractor along the guidewire and catheter and into the patient's body until the distal tip of the retractor contacts the inflated balloon; removing the cap from the body portion of the retractor: separating the two halves of the retractor until the wound is visible: withdrawing the catheter from the patient but leaving the guidewire to guide the wound closure device to the site of the wound; and removing the guidewire and closing the wound. The closing step may preferably comprise either clipping, stapling, and suturing the wound

The method may further comprise the steps of inserting a surgical clip applicator into the retractor body portion after the cap is removed, and advancing the clip applicator over the guidewire and through the channel in the body portion until the applicator contacts the wound. The closing step may preferably comprise applying at least one surgical clip to the wound.

The method may further comprise the step of deflating the balloon and withdrawing the catheter and the guidewire from the patients body prior to the closing step.

In still another preferred embodiment of the present invention, there is disclosed a device to facilitate the closure of wounds in the vasculature of a patient. The device comprises a body portion having an externally threaded proximal end and a tapered distal end. The body portion is separable into two halves, each having a flat internal surface with a groove therein, such that when the internal surfaces abut one another, the grooves form a channel through the body portion. The device also comprises a collar portion distal the externally threaded proximal end comprising at least one guide passage which traverses both halves of said body portion; at least one pin insertable into the guide passage; and an internally threaded annular cap. The internal threads are adapted to engage the external threads on the body portion. The device may also include a handle extending laterally from the pin, and at least one set screw hole in the collar portion at a right angle to the guide passage, and at least one set screw.

The device also comprises a hollow dilator having an open proximal end and an open distal end adapted to receive a guidewire therethrough. The dilator can be inserted through the channel in the body portion and through the annular cap. The dilator further comprises a notch near the distal end sized to receive the tapered distal end of the device. The dilator preferably has at least one indicator hole through a side wall located distal the notch. A pressure sensor can also be mounted on the outside wall of the dilator.

In a preferred embodiment, a guidewire is inserted through the hollow dilator. The hollow dilator has an open proximal end and an open distal end adapted to receive a guidewire therethrough, and the dilator can be inserted through the channel in the body portion and through the annular cap. The hollow dilator may also include a double-sleeved inflatable balloon mounted on its distal end, and at least one indicator hole. The double-sleeved inflatable balloon is mounted approximately 1.5 mm proximal the indicator hole.

The device is preferably comprised of a biocompatible engineering polymer, such as polypropylene, polyethylene, or polyterephthalate, or an elastomer, or a metal, such as stainless steel.

In accordance with yet another aspect of the present invention, there is provided a system for facilitating the closure of wounds in the vasculature of a patient. The system includes a retractor comprising the body portion and the cap defined in claim 1, a hollow dilator adapted to receive a guidewire therethrough, and a guidewire. A guide assembly which is adapted for attachment to the distal end of a surgical clip applicator can also be used. The assembly comprises a guide plate which is reversibly attachable to a guide body having an attached guide tube sized to receive a guidewire. The guide plate has two laterally protruding wings attached, which are adapted to fit within the channel in the retractor.

The system preferably also includes a source of negative pressure attached to the proximal end of the dilator, such as a syringe. A Y-connector having a plurality of ports can also be attached to the proximal end of the dilator, and a source of negative pressure attached to one of the ports of the Y-connector.

There is described a method for facilitating the closure of a wound in the vasculature of a patient. The method may comprise the steps of inserting a proximal end of a hollow dilator having a notch formed near its distal end into a distal end of a retractor comprising a body portion having an externally threaded rounded proximal and, and a tapered distal end; mounting the retractor on the dilator so that the distal tapered end is positioned within the notch on the dilator; inserting a guidewire into the vasculature of the patient through the wound; inserting the proximal end of the guidewire into the distal end of the dilator having the retractor mounted thereon until the proximal end of the guidewire emerges through the proximal end of the dilator; advancing the dilator and retractor along the guidewire and into the patient's body, while providing a source of negative pressure on the dilator until blood is drawn into the dilator from the vasculature; removing the cap from the retractor and separating the two halves of the retractor, removing the guidewire and dilator from the patient's body; and closing the wound.

The wound can be closed by clipping, stapling, or suturing. Preferably, the method may further include the steps of attaching to the distal end of a surgical clip applicator a guide having laterally extending wings, inserting the wings into the retractor body portion and advancing the clip applicator through the channel in the retractor until the applicator contacts the wound. At least one surgical clip is then applied to the wound.

The guide preferably includes a guidetube for receiving the guidewire, and the method further comprises inserting the proximal and of the guidewire through the guidetube. The applicator is advanced over the guidewire and through the channel in the retractor until the applicator contacts the wound

There is described a method for facilitating the closure of a wound in the vasculature of a patient, comprising the steps of inserting a guidewire into the vasculature through the wound until the distal end of the guidewire is within the vasculature and the proximal and remains outside the patient's body; inserting the proximal end of the guidewire into a hollow dilator having a double sleeved balloon mounted on its distal end; advancing the dilator over the guidewire into the vasculature, which is indicated by blood being aspirated through the dilator, which indicates that the wound has been reached; inflating the double-sleeved balloon; inserting the guidewire and dilator into the retractor, advancing the retractor between the two sleeves of the double-sleeved balloon until the distal end reaches the distal detachable junction of the two sleeves; removing the cap from the retractor; separating the two halves of the retractor and removing the dilator and the inner sleeve of the double-sleeved balloon therethrough, leaving the guidewire in place to guide a wound closure device to the wound, the outer sleeve of the balloon forming a tunnel from the surface of the body to the wound; removing the guidewire and closing the wound. The closing of the wound can be done by clipping, stapling, and suturing.

Preferably, the method may include the steps of attaching to a distal end of a surgical clip applicator a guide having laterally extending wings, inserting the wings into the retractor after the cap is removed and advancing the clip applicator through the channel in the retractor until the applicator contacts the wound. At least one surgical clip is then applied to the wound.

The guide preferably further comprises a guidetube for receiving the guidewire, and the method may further comprise inserting the proximal end of the guidewire through the guidetube and advancing the applicator over the guidewire and through the channel in the retractor until the applicator contacts the wound.

The described method may also preferably include providing a source of negative pressure on the dilator until blood is drawn into the dilator from the vasculature, during the advancing of the dilator over the guidewire.

### Brief Description of the Drawings

FIGURE 1 is a side view of a portion of a human body, showing the site where the femoral artery is typically accessed and punctured during angioplasty or angiography.
FIGURE 2 is a perspective view of one embodiment of the wound closure device of the present invention.
FIGURE 3 is an exploded perspective view of the wound closure device of the present invention.
FIGURE 4 is a cross-sectional view of a portion of a human body, showing the femoral artery accessed via a hallow needle, and a guidewire having an inflatable balloon attached, inserted through the hollow needle and into the femoral artery.
FIGURE 5 is a side view of the distal and of a surgical clip applicator to be used in conjunction with the wound closure device of the present invention.
FIGURE 6 is a partial cross-sectional view of a portion of a human body, showing the femoral artery having a guidewire positioned therein, and a perspective view of the retractor of the present invention positioned over the guidewire, with its distal tip at the site of the puncture in the femoral artery.
FIGURE 7 is a side view of the retractor with its cap removed and the wings of the surgical clip applicator inserted into the grooves within the retractor.
FIGURE 8 is a cross-sectional view of the clip applicator and retractor taken along fine 8-8 in FIGURE 7.
FIGURE 9 is a perspective view of an alternate embodiment of a femoral artery closure device in accordance with the present invention.
FIGURE 10 is an exploded perspective view of the alternate embodiment of the femoral artery closure device illustrated in FIGURE 9.
FIGURE 11 is a side view of the 2 halves of the retractor of FIGURES 9 and 10 separated slightly and having a dilator inserted therethrough.
FIGURE 12 is a cross-sectional view of the distal end of the retractor having a dilator and a guidewire inserted therethrough.
FIGURE 13 is a side view of the components of the femoral artery localization and closure assembly.
FIGURE 14 is a side view of the 2 halves of the retractor separated slightly and having a surgical clip applicator with an applicator guide and a guidewire inserted therethrough.
FIGURE 15 is a top view of the surgical clip applicator guide of the present invention.
FIGURE 16 is a side view of the clip applicator guide, having a guidewire inserted therethrough.
FIGURE 17 is an enlarged perspective view of a dilator having a removable double-steoved balloon at its distal end.
FIGURE 18 is an enlarged perspective view of the dilator of FIGURE 17 with the sleeves of the balloon inflated.
FIGURE 19 is an enlarged perspective view of the dilator of FIGURE 18 having an alternate embodiment of a retractor inserted between the sleeves of the balloon.
FIGURE 20 is an enlarged perspective view of the dilator and retractor of FIGURE 19 with the dilator removed, illustrating the tunnel formed by the retractor and the outer sleeve of the balloon.

### Detailed Description of the Preferred Embodiment

### Introduction

Although the description which follows details the closure of a puncture wound in a femoral artery, the present invention is not intended to be limited to use only with the femoral artery. Rather, the description which follows is exemplary only, and those of skill in the art can readily modify the method described below to use with other types of wounds to the vascular system.

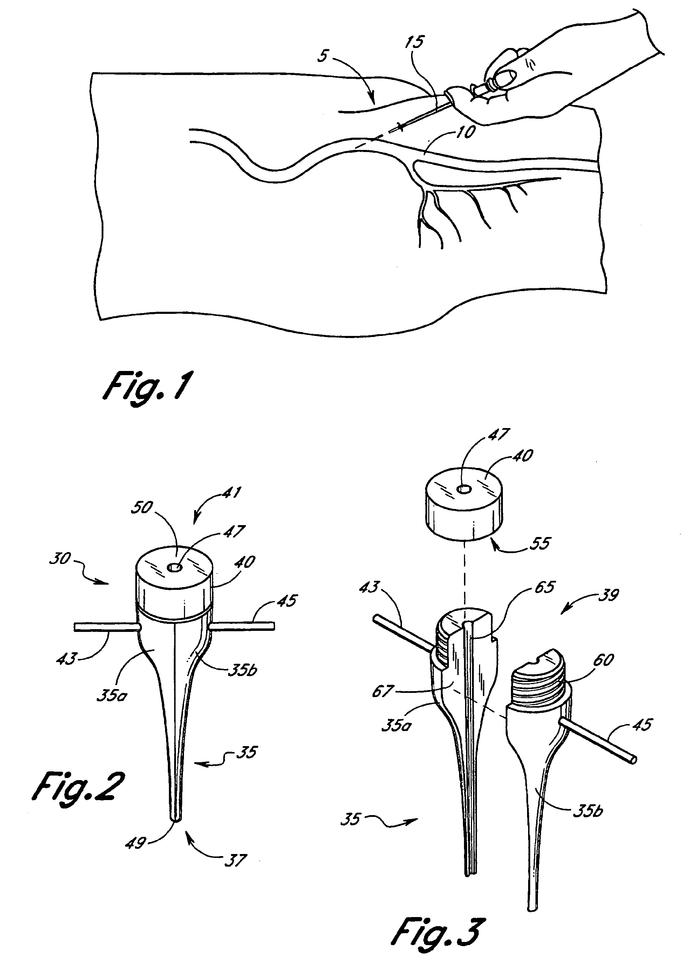

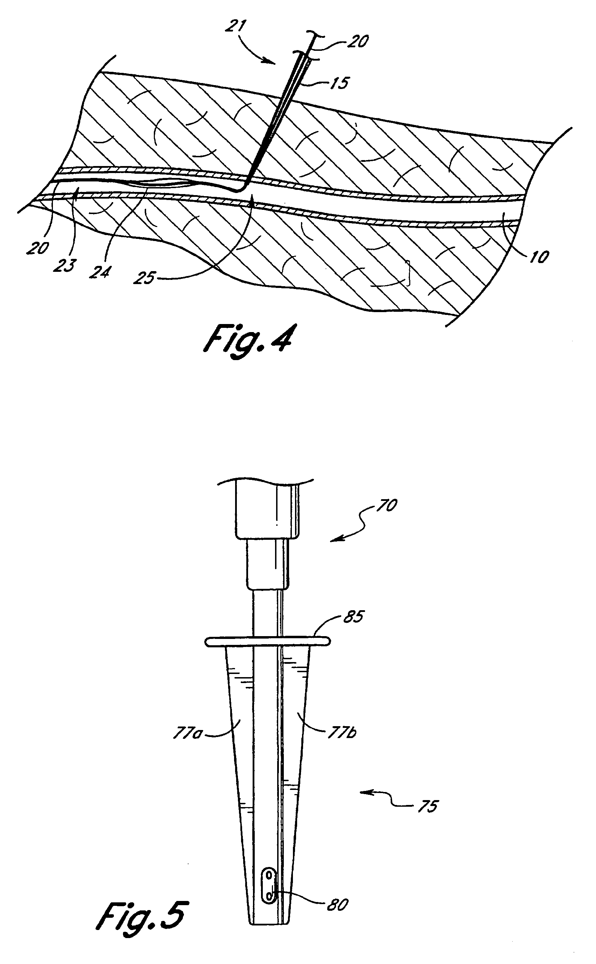

Referring first to FIGURE 1, there is shown a side view of a portion of a human body, showing a site 5 where a femoral artery 10 is typically accessed and punctured during angioplasty or angiography. During these procedures, a hollow needle 15 is first inserted through the skin and into the femoral artery 10. A guidewire 20 is then inserted through the proximal end of the hollow needle 15 and into the artery 10, as illustrated in FIGURE 4, and the needle 15 is withdrawn from the patient. The guidewire 20 is advanced through the patient's vasculature, often using x-ray imaging as an aid in directing the guidewire 20 to the desired location.

Once the guidewire 20 is in the desired location, a catheter is used. The proximal end of the guidewire 21 is inserted into the distal end of the catheter, and the catheter is threaded over the guidewire 20 and advanced to the desired location. In the case of angioplasty, the catheter has an inflatable balloon attached at its distal end. Once in position within the stenosis, the balloon is repeatedly inflated and deflated to widen the narrowed blood vessel. In the case of angiography, a catheter is threaded over the guidewire 20 as just described and into the blood vessel to be examined. Contrast medium is then injected, and a rapid sequence of x-ray pictures are taken so that blood flow along the affected vessel can be studied.

After either of these procedures is completed, the catheter and guidewire 20 are withdrawn from the blood vessel and the patient. The puncture wound 25 in the femoral artery 10 caused by the insertion of the hollow needle 15, guidewire 20 and catheter must be closed and the bleeding through the puncture site 25 in the artery 10 stopped.

### Construction of the Retractor

In order to facilitate the closure of the wound 25 in the femoral artery 10, a retractor 30 is employed. The retractor 30, illustrated in FIGURES 2 and 3, comprises a body portion 35 and a cap 40. The body 35 of the retractor 30 has a narrow, tapered distal end 37, and a broader circular proximal end 41. The device 30 has two handles 43, 45 located on its body 35, one on each half 35a, 35b. The handles 43, 45 are positioned approximately one-third of the way from the proximal end of the retractor 41, and extend laterally from the body of the retractor 35. These handles 43, 45 assist the user in handling the device 30. The retractor 30 also comprises a circular cap 40 at its proximal end 41, having a hole 47 therethrough. This hole 47 extends into a channel 50 which runs the entire length of the device 30.

As illustrated in FIGURE 3, the cap 40 and body 35 of the retractor 30 comprise three separable pieces: the cap portion 40 and the two halves of the body portion 35a, 35b. The removable cap 40 is infernally threaded 55. The proximal end 39 of the two halves of the body 35a, 35b are externally threaded 60, and are adapted to removably receive the cap 40. Each half of the body of the retractor 35a, 35b has a semi-circular groove 65 on its flat internal surface 67. When the cap 40 is securely screwed onto the two halves of the body 35a, 35b as illustrated in FIGURE 2, the three pieces are joined together, and the semi-circular grooves 65 form a channel 50 running through the interior of the device 30, which starts at the hole in the cap 47 at the proximal end 41 and continues through the body 35, ending at a small hole 49 in the distal end of the retractor 37 where the two halves of the body 35a, 35b come together. When the cap 40 is unscrewed from the body 35, the two halves of the body 35a, 35b may be moved apart from one another, as illustrated in FIGURE 3.

### Alternate Embodiment of the Retractor

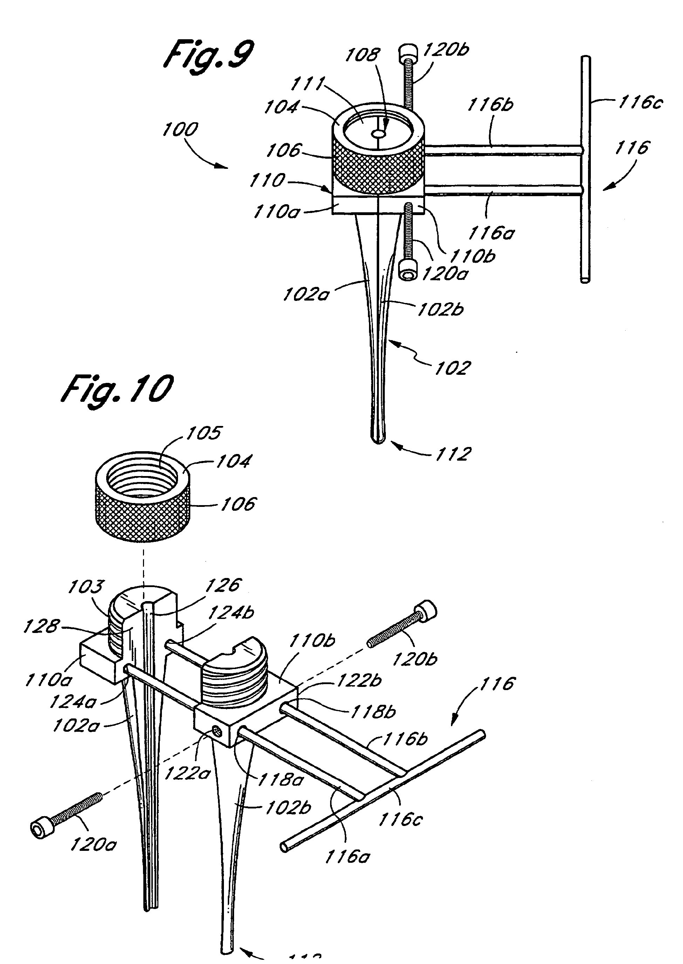

Another preferred embodiment of the invention is illustrated in FIGURES 9-10. In this embodiment, the retractor 100 includes a retraction mechanism whereby the two halves 102a, 102b of the retractor body 102 can be moved apart from one another a desired distance, while maintaining their alignment. The retractor again comprises a body portion 102, and an annular cap 104. The two halves 102a, 102b of the body are initially held together by the internally threaded 105 cap 104. This cap 104 is screwed on and off the externally threaded halves 102a, 102b of the retractor body. The outer surface of the cap 106 can be textured to ease hand tightening and loosening of the cap 106. As illustrated in FIGURE 10, each half 102a, 102b of the retractor body again has a semicircular groove 126 running longitudinally down the center of its flat internal surface 128. When the cap 104 is securely screwed onto the two halves 102a, 102b of the retractor body, such that the internal surfaces 128 abut one another, the semicircular grooves 126 form a channel 108. The cap 104 is open on both ends and through its center to permit access to the channel 108.

The retractor 100, as illustrated in FIGURES 9-10, further comprises a collar 110 located on the retractor body 102 just distal to the externally threaded proximal end 103; a pin assembly 116, comprising two parallel pins 116a, 116b attached at one end to a perpendicular handle 116c and at the other end to one half of the retractor body 102a at points 124a and 124b; and two set screws 120a, 120b. As illustrated in FIGURE 10, the pins 116a, 116b traverse guide passages 118a, 118b bored through the collar region 110b of one half 102b of the retractor body, such that one half 102b of the retractor body can slide apart from the other half 102a on the pins 116a, 116b. The collar 110b includes internally threaded holes 122a, 122b adapted to receive externally threaded set screws 120a, 120b. The set screw holes 122a, 122b enter the collar region 110a at right angles to the pin guide passages 118a, 118b, such that when the set screws 120a, 120b are advanced, they tighten upon the pins 116a, 116b and thus, fix the distance between the two halves 102a, 102b of the retractor body.

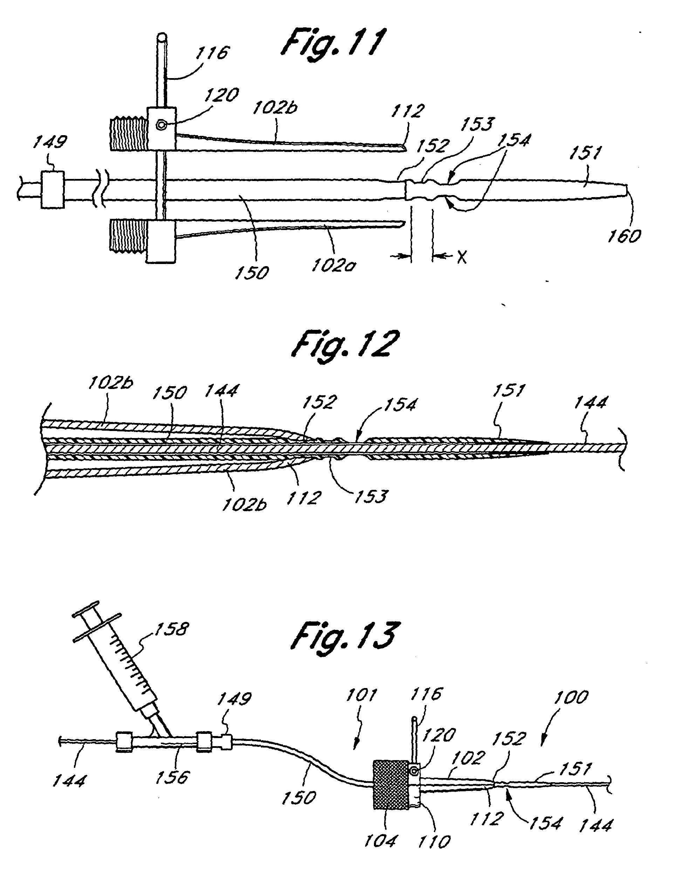

As illustrated in FIGURES 11-13, the retractor 100 is preferably used in conjunction with a dilator 150. As is known to those of ordinary skill in the art, the hollow dilator 150 preferably includes a standard male connector 149, such as a Luer connector, at its proximal end and is narrowly tapered at its distal end 151. The inside diameter of the dilator channel 160 is large enough to accommodate a guidewire 144, so that the dilator 150 can be fed along the guidewire 144 and into the lumen of the femoral artery. Dilators are commonly used in procedures such as angioplasty and angiography to enlarge the puncture site and provide improved access to the femoral artery. In accordance with the present invention, the dilator is preferably notched 152, 154 near its distal end 151 around its entire circumference. Notch 152 provides a seat for the tapered distal tips of the two halves 102a, 102b of the retractor body, such that when the retractor 100 is closed upon the dilator 150, the sharp distal tip of the retractor body 112 is buried in the notch 152 of the dilator. This forms a smooth transition between the dilator 150 and retractor 100 (FIGURE 12). Notch 154 provides a groove in which the wall of the femoral artery rests when the dilator has been properly positioned. As will be explained more fully below, when the guidewire 144 is inserted through the dilator 150 and the dilator 150 is then inserted through the retractor 100, (FIGURES 12-13), the dilator 150 lies securely within the interior circular channel 108 (FIGURE 9) running the length of the retractor body 102.

The dilator 150 also preferably includes at least one indicator hole 154. The dilator 150 illustrated in FIGURES 11-13 includes two indicator holes 154 (FIGURES 11-12), directly opposed to one another, located a few millimeters distal to the notch 152. The distance X between the holes 154 and the notch 153 is preferably only slightly larger than the thickness of the wall of the femoral artery. Alternatively, a transducer-tipped pressure monitoring catheter, mounted to the outside of the dilator 150, may be used in conjunction with the dilator 150 and indicator holes 154. Use of the indicator holes 154 and pressure sensor wall be described in detail below.

A preferred embodiment of the present invention comprising an entire femoral artery localization and closure assembly is illustrated in FIGURE 13. The guidewire. 144 which emerges from the original puncture wound is fed through the dilator 150, and then the dilator 150 is inserted through the retractor 100. The retractor 100 is mounted on the dilator 150 such that the distal tips of the retractor 112 rest within the notch 152 in the dilator 150. Preferably, the male fitting 149 on the proximal end of the dilator 150 is connected to one port of a commercially available 3-way Y-connector 156. A syringe 158 or other means of applying negative pressure is connected to one of the other ports on the Y-connector 156 and the proximal end of the guidewire 144 exits the Y-connector 156 via the remaining port. The Y-connector 156 therefore acts as a seal at the proximal ends of dilator 150 and guidewire 144.

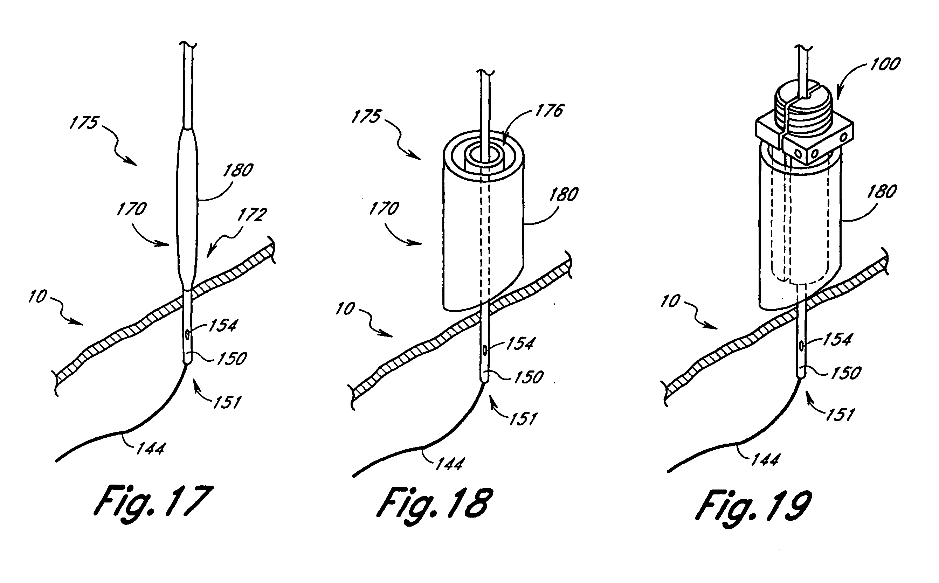

In an alternate preferred embodiment of the invention, a modified dilator and retractor are used. As illustrated in FIGURE 17, a double-sleeved balloon 170 is removably attached to the dilator 150 near its distal end 151, proximal the indicator hole 154. Preferably, the balloon 170 is placed a distance from the indicator hole 154 which is approximately the width of the arterial wall, e.g., 1.5 mm. The inflatable, double-sleeved balloon 170 is angled at its distal end 172 to allow the balloon to fit the femoral artery 10. The balloon 170 includes inflation means which allow the balloon to be inflated and deflated. The retractor 100, rather than having a tapered distal tip as described above, has a cylindrical distal end. This shape prevents the retractor 100 from entering the artery during insertion. Use of the double-sleeved balloon 170 and the cylindrical retractor 100 will be described in detail below.

The retractor of the present invention is preferably formed of one of many strong, biocompatible engineering polymers. Plastics such as polypropylene, polyethylene, or polyterephthalate, are preferred. Elastomers such as silastics or silicones can also be used. Most preferably, metals such stainless or surgical steel, or titanium are used to form the retractor.

### The Surgical Clip Applicator

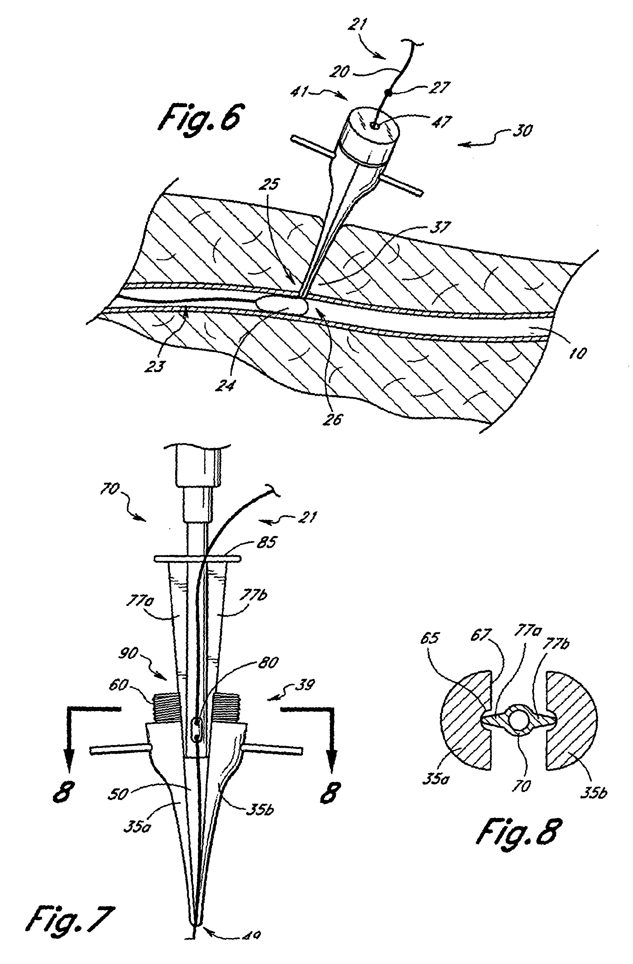

The retractor of the present invention is used to facilitate closure of wounds to the vasculature of a patient using surgical clips, staples, and sutures. One aspect of the present invention therefore includes the use of a surgical clip applicator 70. A surgical clip applicator 70 for use with the retractor 30 of the present invention is illustrated in FIGURE 5. As shown in this figure, the distal end of the clip applicator 75 is fitted with two triangular protrusions or wings 77a, 77b that extend laterally from the sides of the distal end of the clip applicator 75. These wings 77a, 77b are configured to fit within the grooves 65 located on the interior surface of the two halves 35a z 35b of the body of the retractor 30, as is best seen in FIGURE 8. With the wings 77a, 77b of the clip applicator 70 in the grooves 65 in the two halves of the body of the retractor 35a, 35b, the clip applicator 70 is guided into proper position within the patient's body, as will be discussed in more detail below. In addition, the surgical clip applicator 70 preferably has a guide 80 attached to its distal end 75. The guide 80 preferably extends laterally from the side of the clip applicator 70, and is open at its proximal and distal ends such that a guidewire 20 may be threaded therethrough. This guide 80 is used in combination with the guidewire 20 to accurately guide the clip applicator 70 to the site of the vascular puncture 25, as will be described below.

The surgical clip applicator 70 preferably also has a stop 85 located proximal of the distal end 75, at the point where the proximal ends of the wings of the applicator 77a, 77b end. As will be explained, the stop 80 also aids in the proper positioning of the clip applicator 70 at the site of the vascular puncture 25, and prevents the clip applicator 70 from being inserted too far into the patient's body.

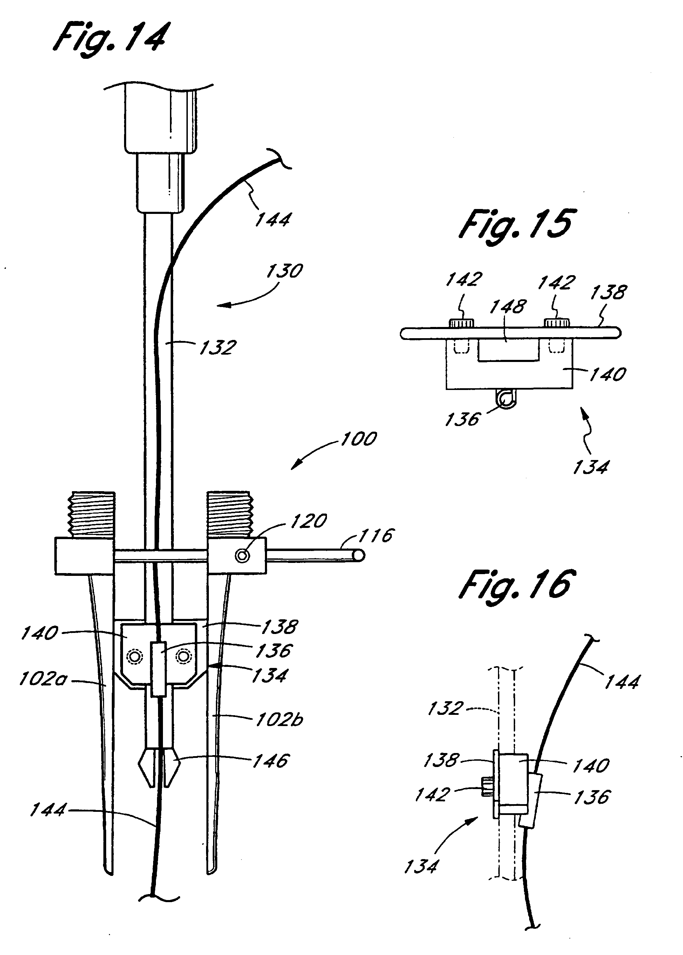

Referring now to FIGURES 14-16, there is illustrated an alternate preferred embodiment of a surgical clip applicator assembly 130. The clip applicator assembly 130 incorporates a standard commercially available surgical clip applicator 132. In accordance with the present invention, the applicator is modified to include a guide assembly 134 reversibly fastened near its distal end. The guide assembly comprises a winged guide plate 138 which is reversibly secured to a body 140. In the embodiment illustrated in FIGURES 14-16, allen screws 142 are used to attach the guide plate 138 but other well known means of attachment can also be used. The distal end of the surgical clip applicator 132 slides within the channel 148 (FIGURE 15) formed when the winged guide plate 138 is fastened to the guide body 140.

Attached to the guide body 140 is a guidetube 136 which is adapted to accept the guidewire 144. A preferred embodiment of said guidetube 136 includes a mechanism to close the guidetube 136 once the guidewire 144 has entered. Such a mechanism may involve a second partially open tube which fits within said guidetube 136. This second tube can be rotated within the guidetube 136 to open the guidetube 136 when the openings in both tubes are aligned or close the guidetube 136 when the openings of the tubes are offset. To facilitate the opening and closing, the inner tube preferably includes a handle that passes through a slot in the outer guidetube 136. This mechanism can be spring-loaded like the closures commonly used on pieces of jewelry.

The surgical clip applicator guide assembly 134, together with the retractor 100 and the guidewire 144, is designed to accurately guide the clip applicator 132 to the site of the femoral artery puncture as detailed below. As explained above, the lateral edges of the winged guide plate 138 are configured to fit within the groove 126 (FIGURE 10) located on the interior surface of each half of the retractor body 102a, 102b. The surgical clip applicator 132 is guided between the retracted halves of the retractor body 102a,102b following the guidewire 144 which passes through the guidetube 136 at the distal most and of the surgical clip applicator 132.

### Method of Use

Referring first to FIGURES 4-8, a method of use of the retractor 30 in conjunction with a surgical clip applicator 70 to close a wound 25 in the femoral artery 10 will now be described. As noted above, during angioplasty or angiography, the femoral artery 10 is first punctured with a hallow needle 15 and a guidewire 20 is inserted therethrough (FIGURE 4). A proximal portion of the guidewire 21 remains outside the patient's body. After the distal end of the guidewire 23 is in position within the femoral artery 10, the hollow needle 15 is removed. A catheter (not shown) is then threaded over the guidewire 20, and inserted into the patient's body.

A specially designed guidewire 20 having an inflatable balloon 24 may be located near its distal end 23 is used for the diagnostic or therapeutic procedure. The guidewire 20 is threaded through the hallow needle 15 and into the patient's vasculature. Alternatively, such as for balloon angioplasty procedures, a standard guidewire well known to those of skill in the art can be used in conjunction with a balloon catheter. The balloon on the distal end of the catheter can be used in place of the balloon 24 located on the guidewire 20.

Following completion of the therapeutic or diagnostic procedure, the catheter used during the procedure is removed. The guidewire 20 remains in place in the patient's vasculature. (Note that when a balloon catheter is used in place of a guidewire having a balloon on its distal end, the catheter is left inside the patient, and use of its balloon is identical to the use of the balloon 24 on the guidewire 20 described below).

When the physician desires to close the wound 25 in the femoral artery 10, he or she first withdraws the guidewire 20 and/or catheter through the patient's vasculature using the portion of the guidewire 20 and/or catheter that remains outside the patient's body 21, until the distal end 23 of the guidewire 20 and/or catheter is within the femoral artery 10 close to the femoral artery puncture site 25. The balloon 24 on the distal end 23 of the guidewire 20 or catheter is then inflated, and the guidewire 20 or catheter is withdrawn further until the physician feels some resistance. This will indicate that the balloon 24 is inside the femoral artery 10 and at the site of the puncture wound 25. The physician then threads the proximal end of the guidewire 21 into the hole 49 located at the distal end 37 of the fully assembled retractor 30 (FIGURES 2, 3 and 6). The guidewire 20 is threaded through the channel 50 formed in the body of the retractor 35, until the proximal end of the guidewire 21 emerges through the hole 47 in the cap 40 at the proximal end of the retractor 41 (FIGURE 6). The retractor 30 is then slowly advanced along the guidewire 20 and into the patient's body, until resistance is felt. This resistance indicates that the distal tip of the retractor 37 is contacting the inflated ballon 24 in the femoral artery 10. The distal tip of the retractor 37 therefore will be property located at the site of the puncture in the femoral artery 25, as is shown in FIGURE 6.

In a preferred embodiment, the guidewire 20 used in conjunction with the femoral artery closure retractor 30 has a marking 27 on it which also helps to indicate when the retractor 30 has been properly positioned (FIGURE 6). This marking 27 preferably consists of a tiny beed or colored line on the guidewire 20. The marking an the guidewire 27 is placed proximal of the proximal and of the ballon 26. The length of the retractor 30 is measured, and the marking 27 is made at least that same length in a proximal direction on the guidewire 20, measured from the proximal end of the balloon 26. Thus, when the retractor 30 is advanced over the guidewire 20 end resistance is felt, the physician checks to see if the marking on the guidewire 27 has emerged through the proximal end of the retractor 41, as is illustrated in FIGURE 6. If the marking 27 is not yet visible, the physician must advance the retractor 30 further to ensure that it contacts the femoral artery puncture site 25.

Once the retractor 30 is properly positioned within the patient's body, the surgical clip applicator 70 or other method of closing the puncture wound 25 is used. The cap 40 on the retractor 30 is first removed from the body by unscrewing (FIGURE 3). The proximal end of the guidewire 21 emerging from the proximal end of the retractor 41 is threaded through the guide 80 located on the outer surface of the applicator 70, as illustrated in FIGURE 7. The wings on the surgical clip applicator 77a, 77b are inserted into the hole 90 formed at the proximal end of the body of the retractor 39, by fining up the wings 77a, 77b on the applicator 30 with the grooves 65 located on the inner surface 67 of the retractor body halves 35a, 35b (FIGURES 7 and 8). The wings on the clip applicator 77a, 77b are sized to fit within the grooves 65 of the retractor 30, as is best illustrated in FIGURE 8. The clip applicator 70 is then advanced, which causes the two halves of the body of the retractor 35a, 35b to separate, as shown in FIGURE 7. As the two halves 35a, 35b separate, the patient's tissue is displaced laterally, allowing better access to the puncture site 25 in the femoral artery 10 below the overlying tissues. The clip applicator 70 is advanced through the retractor 30 until the stop on the applicator 85 contacts the proximal end of the retractor 39. At this time, the balloon on the guidewire 24 or catheter is deflated, and the catheter and/or guidewire 20 is removed from the patient. The surgical dips located at the distal tip of the clip applicator 75 are applied to the puncture wound 25, using the method well known to those of ordinary skill in the art. Once the femoral artery puncture wound 25 is closed, the clip applicator 70 and retractor 30 are removed from the patient.

Referring now to FIGURES 9-16, a method of using the alternate embodiment of the retractor 100 in conjunction with the dilator 150 and surgical clip applicator assembly 130 to localize and close the femoral artery puncture wound is now described. As described above, following completion of the angioplasty or angiography, the catheter used during the procedure is removed from the patient's body, leaving only the guidewire threaded into the femoral artery. If desired, before the retractor-dilator assembly 101 (FIGURE 13) is used, a standard dilator of a smaller diameter than that 150 incorporated into the retractor-dilator assembly 101 can be fed onto the proximal end of the guidewire and advanced down the guidewire and into the artery. This preliminary step dilates the overlying tissue if necessary, making it easier to subsequently pass the larger retractor-dilator assembly 101 through the surrounding tissue.

If the tissue has been dilated as above, the smaller bore standard dilator is first removed. The proximal end of the guidewire 144 is first inserted into the distal channel 160 (FIGURE 11) of the dilator 150. The dilator 150 has been previously inserted through the internal channel of the retractor 100, and the retractor 100 mounted on the dilator 150 such that the distal tip 112 comes to rest in the notch 152 on the distal tip of the dilator 150. The Y-connector 156 is then attached to the proximal end of the dilator 150 and a syringe 158 attached to one of the ports of the connector 156. The retractor-dilator assembly 101 is then advanced over the guidewire 144 into the patient's body.

While the retractor-dilator assembly 101 is advanced into the patient's body, suction is continuously applied via the syringe 158 or other means of negative pressure (FIGURE 13) to the dilator 150. At the moment the indicator holes 154 enter the lumen of the femoral artery, blood is aspirated into the syringe 158, indicating that the dilator 150 has been inserted through the puncture site into the femoral artery. Thus, the distal tip of the retractor 112, still buried within the notch 152 in the dilator 150, is located just proximal or outside the artery wall at the site of the puncture wound and the indicator holes 154 in the dilator 150 are located just distal or inside the artery lumen.

Alternatively, the dilator 150 includes a pressure sensor (not shown) such as a fiber optic pressure sensor, near its distal tip. The sensor is preferably mounted to the outside wall of the dilator 150. In a preferred embodiment, a transducer-tipped pressure monitoring catheter, such as the Camino Catheter available from Camino Laboratories, San Diego, CA, is used. The pressure sensor, mounted on the outside of the dilator 150, is inserted over the guidewire 144 and into the femoral artery. The pressure sensor, in conjunction with a pressure monitoring system, will indicate an increase in pressure when it is inserted into the femoral artery. At that point, the advancement of the retractor 100 is stopped, such that the distal tip of the retractor 112 is located just proximal the artery wall 10 at the site of the puncture wound. This allows the physician to properly locate the site of the femoral artery puncture wound in the patient.

Once the dilator 150 and retractor 100 are in proper position, the cap 104 is removed from the retractor 100 and the two halves 102a, 102b of the retractor body are separated slightly (FIGURE 10) by loosening the set screws 120a, 120b and sliding the two halves 102a, 102b of the retractor laterally away from one another. This causes the distal tips 112 of two halves 102a, 102b to emerge from the notch 152 in the dilator 150 (FIGURE 11) and straddle the puncture site. The set screws 120a, 120b, are then tightened to hold the two halves 102a, 102b of the retractor 100 in this separated position. While pressing the retractor 100 down against the outer wall of the femoral artery, the dilator 150 is withdrawn, leaving only the retractor 100 and the guidewire 144 in position at the site of the puncture wound in the artery.

To close the wound, the retractor 100 must be retracted far enough to allow the surgical clip applicator assembly 130 to access the puncture site. Upon loosening the set screws 120a, 120b, the two halves 102a, 102b of the retractor are further separated by applying pressure on the retractor pin handle 116c (FIGURES 9-10). When sufficiently retracted, the set screws 120a,120b on the retractor assembly 100 are tightened to maintain the proper distance between the retractor halves. If necessary, a separate retractor, having a thickness suited for sliding within the grooves 126 in each half 102a, 102b of the retractor body, and a width equal to that of the winged guide plate 138 (FIGURE 14) of the surgical clip applicator guide assemble 134, can be used to open the retractor body to the proper distance.

In an alternate preferred embodiment illustrated in FIGURE 17, the modified dilator 150 having a double-sleeved inflatable balloon 170 removably attached to the distal end of the dilator 151, just proximal the indicator hole 154, is used. The inner and outer sleeves of the double sleeved balloon 170 are detachable when moved laterally away from each other. The balloon dilator apparatus 175 is inserted over the guidewire 144 into the patient's body. As described above, as the balloon-dilator apparatus 175 is advanced, negative pressure is applied to the system via the syringe or other source. The advance of the balloon-dilator apparatus 175 is stopped as soon as blood is aspirated. The double-sleeved balloon 170 is then inflated to form a tunnel 176 between the femoral artery puncture wound and the surface of the patient's body, as illustrated in FIGURE 18.

The double-sleeved balloon 170 advantageously prevents the femoral artery closure retractor 100 from entering the femoral artery 10 and damaging it. The cylindrical shape of the retractor's distal end 100 also prevents the retractor from entering the artery 10. Should the deflated balloon 170 be advanced into the femoral artery 10, the process of inflating the balloon 170 will pull the balloon 170 out of the artery 10, thereby safely creating a tunnel 176 used to access the artery 10.

The balloon 170 is preferably angled at its distal end 172 to allow the balloon 170 to "fit" the femoral artery 10, as shown in FIGURES 17-19.

Once the balloon 170 is inflated (FIGURE 18) the retractor 100 is advanced between the two sleeves of the inflated balloon 170. The retractor 100 is positioned such that the distal tip of the retractor 112 reaches the distal, detachable junction of the two sleeves of the balloon 170, as shown in FIGURE 19.

Once the retractor 100 is positioned between the two sleeves of the balloon 170, the two halves of the retractor 102a, 102b are moved laterally away from one another, as described above, which causes the two sleeve 1s of the balloon 170 to detach. The inner sleeve 178 and the dilator 150 are removed from the patient, leaving the separated retractor 100 and the outer sleeve 180 of the balloon 170 in the patient.

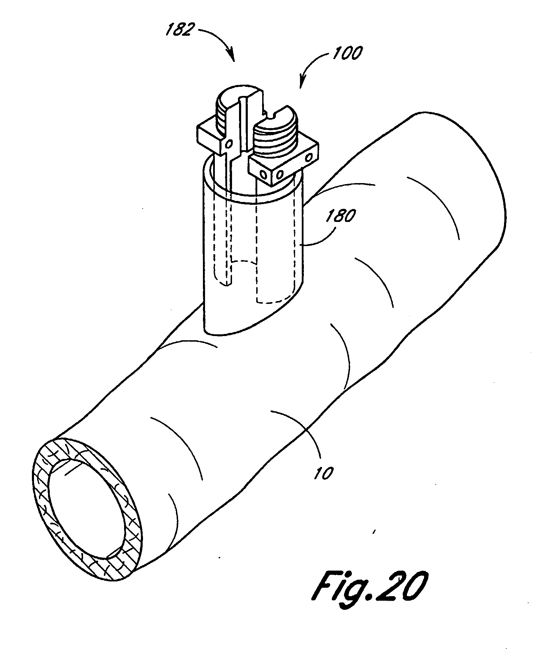

The retractor 100 and the outer sleeve of the balloon 180 form an access tunnel 182 between the femoral artery puncture wound and the surface of the patient's body, as illustrated in FIGURE 20. This tunnel 182 allows for the introduction of the wound closure device to seal the femoral artery puncture wound. The wound closure device can be inserted over the guidewire 144, which acts to guide the device to the site of the wound.

At this point, with the retractor providing access to the femoral artery, the proximal end of the guidewire 144 is inserted into the guidetube 136 on the surgical clip applicator assembly 130 and the wings on the guide plate are fitted within the grooves 126 of the opened retractor body 102 (FIGURES 14-16). The clip applicator assembly 130 can now be advanced toward the puncture wound, sliding within the grooves 126 in the retractor body 102, guided by the guidewire 144 passing through the guidetube 136 at the distal tip of the surgical clip applicator assembly 130. When the distal tip of the surgical clip applicator 130 has reached the outer wall of the femoral artery 10, at the site of the puncture wound, the surgeon withdraws the guidewire 144 from the patient's body and immediately deploys a surgical clip. A second clip can then be deployed a millimeter or two away from the first clip in order to ensure that the wound is closed.

Just prior to closure of the puncture site, the flexible guidewire 144 used during the primary procedure may be replaced with a commercially available variable stiffness guidewire that can become rigid at it's distal end, forming a hook. The hooked distal end is preferably used to "hook" the bifurcation between the femoral and iliac arteries. Once hooked at the bifurcation, the guidewire cannot be pulled out of the patient's vasculature. Thus, when resistance is felt and the guidewire is hooked at the bifurcation, the guidewire is pulled back further and lifted slightly, causing the puncture wound to stretch into a linear slit and the edges of the wound are brought together, making it more amenable to closure by surgical clips.

The retractor can also be used with surgical staples or sutures. After the retractor is inserted into the patient's body and positioned within the puncture site as described above, the two halves of the retractor are manually separated, literally displacing the tissues surrounding the puncture site 25. The retractor acts much like a dilator, gradually increasing the displacement of the overlying tissues, until the puncture wound is visible to the physician. The wound can then be closed using any acceptable means for wound closure, including surgical staples and sutures.

Although certain embodiments and examples have been used to illustrate and describe the present invention, it is intended that the scope of the invention not be limited to the specific embodiments set forth herein. The scope of the invention is to be defined by the claims which follow.

## Claims

1. A device (30, 100) to facilitate the closure of wounds in the vasculature of a patient, comprising:
a body portion (35, 102) having an externally threaded proximal end and a distal end (37, 112), wherein said body portion is separable into two halves (35a, 35b, 102a, 102b), each of said halves having a flat internal surface with a groove (65, 126) therein, such that when said internal surfaces abut one another, said grooves form a channel (50, 108) through said body portion;
an internally threaded cap (40, 104), said internal threads adapted to engage said external threads on said body portion; a hollow dilator (150) having an open proximal end and an open distal end adapted to receive a guidewire therethrough, wherein said dilator can be inserted through said channel in said body portion , wherein said dilator has at least one indicator hole through a sidewall thereof,
wherein either:
1. the distal end of said body portion is tapered, the cap has a hole therethrough and the dilator can be inserted through the hole in the cap;
said internal threads adapted to engage said external threads on said body portion such that when said cap engages said body portion, said hole is positioned directly above said channel in said body portion;
2. the device comprises a collar portion (110) distal the externally threaded proximal end comprising at least one guide passage (118a, 118b) which traverses one half of said body portion;
at least one pin (116a, 116b) extending from one half of said body portion and insertable into said guide passage in the other half of said body portion;
said cap is an internally threaded annular cap (104); and said dilator can be inserted through said annular cap;

2. The device of claim 1, further comprising at least one handle (43, 45, 116c) located on said proximal end of said body portion and extending laterally from said body portion.

3. The device of claim 1 or 2, wherein said body portion and said cap are comprised of a biocompatible engineering polymer.

4. The device of claim 3, wherein said polymer is selected from the group consisting of polypropylene, polyethylene, or polyterephthalate.

5. The device of claim 1 or 2, wherein said body portion and said cap are comprised of an elastomer.

6. The device of claim 1 or 2, wherein said body portion and said cap are comprised of a metal.

7. The device of claim 1 or 2, wherein said body portion and said cap are comprised of stainless steel.

8. The device of any one of claims 1 to 6, further comprising a guidewire (20), wherein said guidewire can be inserted through said channel in said body portion and through said hole in said cap.

9. The device of claim 8, wherein said guidewire has an inflatable balloon (24) attached thereto.

10. A system for facilitating the closure of wounds in the vasculature of a patient, comprising:
a retractor comprising the body portion and the internally threaded cap of any one of claims 1 to 6; a guidewire (20) having an inflatable balloon (24) attached thereto, wherein said guidewire can be inserted through said channel in said body portion and through said hole in said cap; and
a surgical clip applicator (70, 130) having a distal end, wherein said distal end of said applicator has two laterally protruding wings (77, 138) attached, said wings being adapted to fit within the channel in said body portion of said retractor.

11. The system of claim 10, wherein said surgical clip applicator further comprises a guide (80, 136) attached to said distal end of said applicator and extending laterally therefrom, wherein said guide is adapted to receive said guidewire therethrough.

12. The device of claim 1 according to option 2, further comprising a handle (116c) extending laterally from said pin.

13. The device of claim 1 according to option 2, further comprising at least one set screw hole (122a, 122b) in said collar portion at a right angle to said guide passage, and at least one set screw insertable into said set screw hole.

14. The device of any one of claims 1 to 6 according to option 2, wherein said distal end is cylindrical.

15. The device of any one of claims 1 to 6 and 14, wherein said distal end is tapered and wherein said dilator further comprises a notch (153) near said distal end sized to receive the tapered distal end of the body portion of the device.

16. The device of claim 15, wherein said at least one indicator hole is located distal said notch.

17. The device of any one of claims 1 to 6,wherein said dilator further comprises a pressure sensor mounted on an outside wall of said dilator.

18. The device of any one of claims 1 to 6, further comprising a guidewire (144), wherein said guidewire can be inserted through said hollow dilator.

19. The device of any one of claims 1 to 6, wherein said hollow dilator has a double-sleeved inflatable balloon (170) mounted on its distal end.

20. The device of claim 19, wherein said at least one indicator hole (154) is located at a distal end, and wherein said double-sleeved inflatable balloon is mounted approximately 1.5 mm proximal said indicator hole.

21. The device of claim 1 according to option 2, further comprising a guide assembly (134) adapted for attachment to a distal end of a surgical clip applicator (130), said assembly comprising a guide plate (138) which is reversibly attachable to a guide body (140) having an attached guide tube (136) sized to receive a guidewire therethrough, wherein said guide plate has two laterally protruding wings attached, said wings being adapted to fit within the channel in said body portion of said retractor.

22. The device of claim 1, 18 or 21, further comprising a source (158) of negative pressure attached to said open proximal end of said dilator.

23. The device of claim 22, wherein said source of negative pressure comprises a syringe.

24. The device of claim 1, 17 or 21, further comprising a Y-connector (156) having a plurality of ports attached to the proximal end of the dilator at one of said ports.

25. The device of claim 24, further comprising a source (158) of negative pressure attached to one of the ports of said Y-connector.

26. The device of any one of claims 1 to 6, wherein the dilator comprises a seat for the distal end of the two halves of the body portion.

27. The device of claim 26, wherein the seat comprises a notch.

28. The device of claim 26 or 27, wherein the at least one indicator hole is located distal said seat.

29. The device of any one of claims 1 to 6, wherein a distal end of the body portion is located proximal of the at least one indicator hole.

## Patentansprüche

1. Vorrichtung (30, 100) zum Erleichtern des Verschließens von Wunden in den Blutgefäßen eines Patienten, umfassend:
einen Körperabschnitt (35, 102) mit einem mit Außengewinde versehenen proximalen Ende und einem distalen Ende (37, 112), wobei der Körperabschnitt in zwei Hälften (35a, 35b, 102a, 102b) trennbar ist, wobei jede der Hälften eine flache Innenoberfläche mit einer Nut (65, 126) darin aufweist, so dass die Nuten, wenn die Innenoberflächen aneinander anliegen, einen Kanal (50, 108) durch den Körperabschnitt bilden;
eine mit Innengewinde versehene Kappe (40, 104), wobei die Innengewinde dazu angepasst sind, die Außengewinde an dem Körperabschnitt zu ergreifen; einen hohlen Dilator (150) mit einem offenen proximalen Ende und einem offenen distalen Ende, das dazu angepasst ist, einen Führungsdraht durch sich hindurch aufzunehmen, wobei der Dilator durch den Kanal in den Körperabschnitt eingeführt werden kann,
wobei der Dilator zumindest eine Indikatoröffnung durch eine Seitenwand von ihm aufweist, wobei entweder:
1. sich das distale Ende des Körperabschnitts verjüngt, die Kappe eine Öffnung durch sich aufweist und der Dilator durch die Öffnung in der Kappe eingeführt werden kann;
wobei die Innengewinde dazu angepasst sind, die Außengewinde an dem Körperabschnitt derart zu ergreifen, dass, wenn die Kappe den Körperabschnitt ergreift, die Öffnung unmittelbar oberhalb des Kanals in dem Körperabschnitt positioniert ist; oder
2. die Vorrichtung einen Kragenabschnitt (110) distal des mit Außengewinde versehenen proximalen Endes aufweist, der zumindest einen Führungsdurchgang (118a, 118b) aufweist, der eine Hälfte des Körperabschnitts durchquert;
zumindest einen Stift (116a, 116b), der sich von einer Hälfte des Körperabschnitts erstreckt und in den Führungsdurchgang in der anderen Hälfte des Körperabschnitts einführbar ist;
wobei die Kappe eine mit Innengewinde versehene ringförmige Kappe (104) ist; und wobei der Dilator durch die ringförmige Kappe eingeführt werden kann.

2. Vorrichtung nach Anspruch 1, ferner umfassend zumindest einen Griff (43, 45, 116c), der an dem proximalen Ende des Körperabschnitts angeordnet ist und sich seitlich von dem Körperabschnitt aus erstreckt.

3. Vorrichtung nach Anspruch 1 oder 2, wobei der Körperabschnitt und die Kappe aus einem biokompatiblen technischen Polymer sind.

4. Vorrichtung nach Anspruch 3, wobei das Polymer aus der Gruppe ausgewählt ist, die aus Polypropylen, Polyethylen oder Polyterephthalat besteht.

5. Vorrichtung nach Anspruch 1 oder 2, wobei der Körperabschnitt und die Kappe aus einem Elastomer bestehen.

6. Vorrichtung nach Anspruch 1 oder 2, wobei der Körperabschnitt und die Kappe aus einem Metall bestehen.

7. Vorrichtung nach Anspruch 1 oder 2, wobei der Körperabschnitt und die Kappe aus Edelstahl bestehen.

8. Vorrichtung nach einem der Ansprüche 1 bis 6, ferner umfassend einen Führungsdraht (20), wobei der Führungsdraht durch den Kanal in dem Körperabschnitt und durch die Öffnung in der Kappe eingeführt werden kann.

9. Vorrichtung nach Anspruch 8, wobei der Führungsdraht einen aufblasbaren Ballon (24) aufweist, der an ihm angebracht ist.

10. System zum Erleichtern des Verschließens von Wunden in den Blutgefäßen eines Patienten, umfassend:
einen Retraktor, der den Körperabschnitt und die mit Innengewinde versehene Kappe nach einem der Ansprüche 1 bis 6 aufweist; einen Führungsdraht (20) mit einem aufblasbaren Ballon (24), der an ihm angebracht ist, wobei der Führungsdraht durch den Kanal in dem Körperabschnitt und durch die Öffnung in der Kappe eingeführt werden kann; und
einen chirurgischen Clipapplikator (70, 130) mit einem distalen Ende, wobei an dem distalen Ende des Applikators zwei seitlich vorstehende Flügel (77, 138) angebracht sind, wobei die Flügel dazu angepasst sind, in den Kanal in dem Körperabschnitt des Retraktors zu passen.

11. System nach Anspruch 10, wobei der chirurgische Clipapplikator ferner eine Führung (80, 136), die an dem distalen Ende des Applikators angebracht ist und sich seitlich von ihm erstreckt, aufweist, wobei die Führung dazu angepasst ist, den Führungsdraht durch sie aufzunehmen.

12. Vorrichtung nach Anspruch 1 gemäß Option 2, ferner umfassend einen Griff (116c), der sich seitlich von dem Stift erstreckt.

13. Vorrichtung nach Anspruch 1 gemäß Option 2, ferner umfassend zumindest eine Einstellschraubenöffnung (122a, 122b) in dem Kragenabschnitt in einem rechten Winkel zu dem Führungsdurchgang und zumindest eine Einstellschraube, die in die Einstellschraubenöffnung einführbar ist.

14. Vorrichtung nach einem der Ansprüche 1 bis 6 gemäß Option 2, wobei das distale Ende zylindrisch ist.

15. Vorrichtung nach einem der Ansprüche 1 bis 6 und 14, wobei das distale Ende sich verjüngt und wobei der Dilator ferner einen Einschnitt (153) nahe dem distalen Ende aufweist, der dazu bemessen ist, das sich verjüngende distale Ende des Körperabschnitts der Vorrichtung aufzunehmen.

16. Vorrichtung nach Anspruch 15, wobei die zumindest eine Indikatoröffnung distal von dem Einschnitt angeordnet ist.

17. Vorrichtung nach einem der Ansprüche 1 bis 6, wobei der Dilator ferner einen Drucksensor aufweist, der an einer Außenwand des Dilators angebracht ist.

18. Vorrichtung nach einem der Ansprüche 1 bis 6, ferner umfassend einen Führungsdraht (144), wobei der Führungsdraht durch den hohlen Dilator eingeführt werden kann.

19. Vorrichtung nach einem der Ansprüche 1 bis 6, wobei der hohle Dilator einen doppelwandigen aufblasbaren Ballon (170) aufweist, der an seinem distalen Ende angebracht ist.

20. Vorrichtung nach Anspruch 19, wobei die zumindest eine Indikatoröffnung (154) an einem distalen Ende angeordnet ist und wobei der doppelwandige aufblasbare Ballon in etwa 1,5 mm proximal zu der Indikatoröffnung angebracht ist.

21. Vorrichtung nach Anspruch 1 gemäß Option 2, ferner umfassend eine Führungsanordnung (134), die zur Anbringung an einem distalen Ende eines chirurgischen Clipapplikators (130) angepasst ist, wobei die Anordnung eine Führungsplatte (138) umfasst, die reversibel an einem Führungskörper (140) anbringbar ist, der eine angebrachte Führungsröhre (136) aufweist, die dazu bemessen ist, einen Führungsdraht durch sich aufzunehmen, wobei an der Führungsplatte zwei seitlich vorstehende Flügel angebracht sind, wobei die Flügel dazu angepasst sind, in den Kanal in dem Körperabschnitt des Retraktors zu passen.

22. Vorrichtung nach Anspruch 1, 18 oder 21, ferner umfassend eine Unterdruckquelle (158), die an dem offenen proximalen Ende des Dilators angebracht ist.

23. Vorrichtung nach Anspruch 22, wobei die Unterdruckquelle eine Spritze aufweist.

24. Vorrichtung nach Anspruch 1, 17 oder 21, ferner umfassend einen Y-Verbinder (156) mit mehreren Anschlüssen, die an dem proximalen Ende des Dilators an einem der Anschlüsse angebracht sind.

25. Vorrichtung nach Anspruch 24, ferner umfassend eine Unterdruckquelle (158), die an einem der Anschlüsse des Y-Verbinders angebracht ist.

26. Vorrichtung nach einem der Ansprüche 1 bis 6, wobei der Dilator einen Sitz des distalen Endes der zwei Hälften des Körperabschnitts umfasst.

27. Vorrichtung nach Anspruch 26, wobei der Sitz einen Einschnitt aufweist.

28. Vorrichtung nach Anspruch 26 oder 27, wobei die zumindest eine Indikatoröffnung distal des Sitzes angeordnet ist.

29. Vorrichtung nach einem der Ansprüche 1 bis 6, wobei das distale Ende des Körperabschnitts proximal der zumindest einen Indikatoröffnung angeordnet ist.

## Revendications

1. Dispositif (30, 100) permettant de faciliter la fermeture de plaies dans le système vasculaire d'un patient, comprenant :
une portion de corps (35, 102) comportant une extrémité proximale et une extrémité distale (37, 112) filetées à l'extérieur, où ladite portion de corps est séparable en deux moitiés (35a, 35b, 102a, 102b), chacune desdites moitiés comportant une surface interne plate renfermant une rainure (65, 126), de telle sorte que lorsque lesdites surfaces internes viennent en butée l'une contre l'autre, lesdites rainures forment un canal (50, 108) à travers ladite portion de corps ;
un capuchon fileté à l'intérieur (40, 104), lesdits filetages internes étant adaptés pour coopérer avec lesdits filetages externes sur ladite portion de corps ; un dilatateur creux (150) comportant une extrémité proximale ouverte et une extrémité distale ouverte adaptées pour recevoir un fil guide au travers, où ledit dilatateur peut être inséré à travers ledit canal dans ladite portion de corps, où ledit dilatateur comporte au moins un trou indicateur à travers une paroi latérale de celui-ci,
dans lequel :
1. soit l'extrémité distale de ladite portion de corps est effilée, le capuchon comportant un trou au travers et le dilatateur peut être inséré à travers le trou dans le capuchon :
lesdits filetages internes sont adaptés pour coopérer avec lesdits filetages externes sur ladite portion de corps de telle sorte que lorsque ledit capuchon coopère avec ladite portion de corps, ledit trou est positionné directement au-dessus dudit canal dans ladite portion de corps ;
2. soit le dispositif comprend une portion de collier (110) distale de l'extrémité proximale filetée de manière externe d'au moins un passage guide (118a, 118b) qui traverse une moitié de ladite portion de corps ;
au moins une broche (116a, 116b) s'étendant depuis une moitié de ladite portion de corps et pouvant être insérée dans ledit passage guide dans l'autre moitié de ladite portion de corps :
ledit capuchon est un capuchon annulaire fileté à l'intérieur (104) ;
et ledit dilatateur peut être inséré à travers ledit capuchon annulaire.

2. Dispositif selon la revendication 1, comprenant en outre au moins une poignée (43, 45, 116c) située sur ladite extrémité proximale de ladite portion de corps et s'étendant latéralement depuis ladite portion de corps.

3. Dispositif selon la revendication 1 ou 2, dans lequel ladite portion de corps et ledit capuchon sont composés d'un polymère technique biocompatible.

4. Dispositif selon la revendication 3, dans lequel ledit polymère est choisi dans le groupe constitué par le poly(propylène), le poly(éthylène) ou le poly(téréphtalate).

5. Dispositif selon la revendication 1 ou 2, dans lequel ladite portion de corps et ledit capuchon sont composés d'un élastomère.

6. Dispositif selon la revendication 1 ou 2, dans lequel ladite portion de corps et ledit capuchon sont composés d'un métal.

7. Dispositif selon la revendication 1 ou 2, dans lequel ladite portion de corps et ledit capuchon sont composés d'acier inoxydable.

8. Dispositif selon l'une quelconque des revendications 1 à 6, comprenant en outre un fil guide (20), où ledit fil guide peut être inséré à travers ledit canal dans ladite portion de corps à travers ledit trou dans ledit capuchon.

9. Dispositif selon la revendication 8, dans lequel un ballonnet gonflable (24) est attaché audit fil guide.

10. Système permettant de faciliter la fermeture de plaies dans le système vasculaire d'un patient, comprenant :
un rétracteur comprenant la portion de corps et le capuchon fileté à l'intérieur selon l'une quelconque des revendications 1 à 6 ; un fil guide (20) auquel est attaché un ballonnet gonflable (24), où ledit fil guide peut être inséré à travers ledit canal dans ladite portion de corps et à travers ledit trou dans ledit capuchon ; et
un applicateur de pince chirurgicale (70, 130) comportant une extrémité distale, où ladite extrémité distale dudit applicateur comporte deux ailes faisant saillie latéralement (77, 138) attachées, lesdites ailes étant adaptées pour s'ajuster dans le canal de ladite portion de corps dudit rétracteur.

11. Système selon la revendication 10, dans lequel l'applicateur de pince chirurgicale comprend en outre un guide (80, 136) fixé à ladite extrémité distale dudit applicateur et s'étendant latéralement depuis celle-ci, où ledit guide est adapté pour recevoir ledit fil guide qui le traverse.

12. Dispositif selon la revendication 1 selon l'option 2, comprenant en outre une poignée (116c) s'étendant latéralement depuis ladite broche.

13. Dispositif selon la revendication 1 selon l'option 2, comprenant en outre au moins un trou de vis de pression (122a, 122b) dans ladite portion de collier à angle droit par rapport audit passage guide, et au moins une vis de pression pouvant être insérée dans ledit trou de vis de pression.

14. Dispositif selon l'une quelconque des revendications 1 à 6 selon l'option 2, dans lequel ladite extrémité distale est cylindrique.

15. Dispositif selon l'une quelconque des revendications 1 à 6 et 14, dans lequel ladite extrémité distale est effilée et dans lequel ledit dilatateur comprend en outre une encoche (153) près de ladite extrémité distale, dimensionnée pour recevoir l'extrémité distale effilée de la portion de corps du dispositif.

16. Dispositif selon la revendication 15, dans lequel ledit au moins un trou indicateur est situé à distance de ladite encoche.

17. Dispositif selon l'une quelconque des revendications 1 à 6, dans lequel ledit dilatateur comprend en outre un capteur de pression monté sur une paroi extérieure dudit dilatateur.

18. Dispositif selon l'une quelconque des revendications 1 à 6, comprenant en outre un fil guide (144), où ledit fil guide peut être inséré à travers ledit dilatateur creux.

19. Dispositif selon l'une quelconque des revendications 1 à 6, dans lequel ledit dilatateur creux comporte un ballonnet gonflable à manchon double (170) monté sur son extrémité distale.

20. Dispositif selon la revendication 19. dans lequel ledit au moins un trou indicateur (154) est situé à une extrémité distale, et dans lequel ledit ballonnet gonflable à manchon double est monté à approximativement 1,5 mm dudit trou indicateur.

21. Dispositif selon la revendication 1 selon l'option 2, comprenant en outre un ensemble guide (134) adapté pour une fixation à une extrémité distale de l'applicateur de pince chirurgicale (130), ledit ensemble comprenant une plaque guide (138) qui peut être attachée de façon réversible à un corps guide (140) comportant un tube guide fixé (136) dimensionné pour recevoir un fil guide au travers, où ladite plaque guide comporte deux ailes attachées faisant saillie latéralement, lesdites ailes étant adaptées pour s'ajuster dans le canal dans ladite portion de corps dudit rétracteur.

22. Dispositif selon la revendication 1, 18 ou 21, comprenant en outre une source (158) de pression négative fixée à ladite extrémité proximale ouverte dudit dilatateur.

23. Dispositif selon la revendication 22, dans lequel ladite source de pression négative comprend une seringue.

24. Dispositif selon la revendication 1, 17 ou 21, comprenant en outre un raccord en Y (156) comportant une pluralité d'orifices fixée à l'extrémité proximale du dilatateur au niveau de l'un desdits orifices.

25. Dispositif selon la revendication 24, comprenant en outre une source (158) de pression négative fixée à l'un des orifices dudit raccord en Y.

26. Dispositif selon l'une quelconque des revendications 1 à 6, dans lequel le dilatateur comprend un siège pour l'extrémité distale des deux moitiés de la portion de corps.

27. Dispositif selon la revendication 26, dans lequel le siège comprend une encoche.

28. Dispositif selon la revendication 26 ou 27, dans lequel ledit au moins un trou indicateur est situé à distance dudit siège.

29. Dispositif selon l'une quelconque des revendications 1 à 6, dans lequel une extrémité distale de la portion de corps est située à proximité dudit au moins un trou indicateur.
